# FASCICULE DE BREVET EUROPEEN

(11) **EP 4 016 417 B1**
(45) Date de publication et mention de la délivrance du brevet: **31.12.2025**
(21) Numéro de dépôt: 21214542.9
(22) Date de dépôt: 14.12.2021
(51) Int. Cl.: G06Q 10/0631

(54) **SYSTÈME DE DÉTERMINATION D'UN ÉTAT OPÉRATIONNEL D'UN ÉQUIPAGE D AÉRONEF EN FONCTION D'UN PLAN DE TÂCHES ADAPTATIF ET PROCÉDÉ ASSOCIÉ**
SYSTEM ZUR BESTIMMUNG DES EINSATZZUSTANDS EINER FLUGZEUGBESATZUNG IN ABHÄNGIGKEIT VON EINEM ADAPTIVEN AUFGABENPLAN UND ENTSPRECHENDES VERFAHREN
SYSTEM FOR DETERMINING A WORKING CONDITION OF AN AIRCRAFT CREW ACCORDING TO AN ADAPTIVE TASK PLAN AND ASSOCIATED METHOD

(30) Priorité: 15.12.2020 FR 2013237
(43) Date de publication de la demande: 22.06.2022
(73) Titulaire: Dassault Aviation, 75008 Paris (FR)
(72) Inventeur: DARGENT, Lauren, 92214 SAINT CLOUD (FR); GIROD, Hervé, 92214 SAINT CLOUD (FR)
(74) Mandataire: Lavoix

(56) Documents cités:
- EP-A2- 2 437 033

## Description

La présente invention concerne un système de détermination d'un état opérationnel d'un équipage d'aéronef habité ou non habité, en fonction d'un plan de tâches adaptatif.

EP2437033 décrit un système de suivi de tâches d'un équipage et de réattribution de tâches.

L'état opérationnel d'un aéronef est notamment la capacité d'un équipage à réaliser les tâches de sa mission en fonction de son état courant.

Ce système est destiné notamment à évaluer en temps réel l'état opérationnel de l'équipage, eu égard à l'ensemble des tâches qu'il doit effectuer avec l'aéronef dans le cadre d'une mission. L'aéronef est par exemple habité et l'équipage est présent dans le cockpit de l'aéronef. En variante, l'aéronef est non habité, l'équipage est alors dans une station sol de contrôle de drone.

La mission est par exemple une mission d'un aéronef civil, comme un vol commercial ou privé entre un point géographique de départ et un point géographique d'arrivée, dans lequel, lors des phases de la mission, de nombreuses tâches doivent être effectuées conformément aux procédures applicables.

Dans une variante, la mission est une mission d'un aéronef militaire (avion habité ou drone), comportant en particulier des tâches de reconnaissance ou de combat, par exemple des tâches de combat air-sol pour neutraliser des cibles.

Dans le cadre de telles missions, l'équipage suit généralement un plan de tâches comportant une liste de tâches à effectuer, associées avec le temps auquel la tâche doit être effectuée.

Au cours d'une mission, le plan de tâches est adaptatif. Ceci signifie que la liste des tâches à effectuer est mise à jour de façon automatique par l'aéronef et est validée par l'équipage, notamment en fonction d'évènements se produisant au cours de la mission. L'équipage peut aussi décider de lui-même de modifier le plan de tâches.

Par exemple, les objectifs du plan de mission ou du plan de vol sont susceptibles d'évoluer, notamment lors d'un déroutement pour un aéronef civil, ou lorsqu'une nouvelle cible doit être traitée pour un aéronef militaire.

De même, l'environnement de la mission est susceptible d'évoluer, par exemple en fonction de la météorologie dans les domaines civil ou militaire, ou de l'apparition de menaces supplémentaires dans le domaine militaire.

Dans certains cas, les ressources de l'équipage, c'est-à-dire les capacités de l'équipage à réaliser ses tâches, ne sont pas adaptées au plan de tâches à effectuer.

Ceci peut s'expliquer par exemple parce qu'un grand nombre de tâches additionnelles sont à réaliser, en raison d'une panne ou d'une urgence intervenue sur l'aéronef. L'équipage peut également être fatigué ou stressé, ce qui ne lui permet pas de réaliser les tâches qui lui incombent.

Enfin, dans certains cas critiques, l'équipage de l'aéronef peut se retrouver dépassé par l'ensemble des tâches à effectuer, ou au contraire se concentrer trop sur une tâche particulière, et négliger des tâches importantes qui devraient être accomplies.

Pour vérifier l'état de l'équipage, il est connu d'équiper le cockpit de l'aéronef de capteurs mesurant l'état physiologique de l'équipage. Cependant, ces capteurs s'intéressent juste à l'état instantané d'un membre d'équipage, sans relier cet état instantané avec les contraintes que subit l'équipage pour réaliser le plan de mission.

Un but de l'invention est d'obtenir un système de suivi du comportement de l'équipage lors de l'exécution d'un plan de tâches qui détermine de manière fiable et dynamique l'aptitude de l'équipage à réaliser sa mission.

À cet effet, l'invention a pour objet un système selon la revendication 1.

Le système selon l'invention peut comprendre l'une ou plusieurs des caractéristiques des revendications 2 à 11, ou l'une ou plusieurs des caractéristiques suivantes prise(s) isolément ou suivant toute combinaison techniquement possible :
- le module de détermination d'états cognitifs globaux détermine les états cognitifs globaux également à partir des mesures d'états d'interactions du module de mesures d'états d'interactions ;
- le module de détermination d'états cognitifs locaux détermine les états cognitifs locaux également à partir des mesures d'états d'interactions du module de mesures d'états d'interactions ;
- les états d'interactions sont choisis parmi au minimum un niveau d'attention, un niveau de performance, une stratégie de conduite des tâches ;
- le module de détermination de ressources équipages est propre à affecter, via le modèle de ressources, pour l'ensemble des tâches à effectuer, l'état cognitif global nécessaire pour effectuer les tâches, avantageusement en fonction du profil du membre d'équipage donné par la base de données et des indicateurs de contexte définis par le module de détermination d'indicateurs de contexte ;
- le module de détermination de ressources équipages est propre à affecter, via le modèle de ressources, pour chaque tâche de la liste des tâches l'état cognitif local nécessaire pour effectuer ladite tâche avantageusement en fonction du profil du membre d'équipage.

L'invention a également pour objet un procédé selon la revendication 12.

Le procédé selon l'invention peut comprendre l'une ou plusieurs des caractéristiques des revendications 13 et 14.

L'invention sera mieux comprise à la lecture de la description qui va suivre, donnée uniquement à titre d'exemple, et faite en se référant aux dessins annexés, sur lesquels :
- [Fig 1] la figure 1 est un schéma synoptique illustrant un premier système de détermination de l'état opérationnel d'équipage selon l'invention, intégré au sein d'un aéronef ;

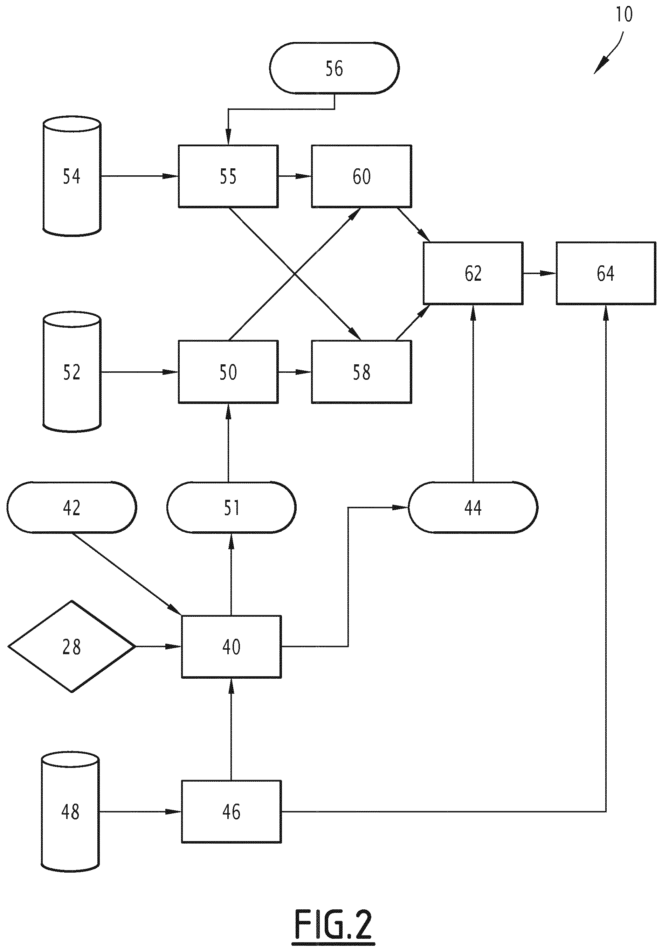

- [Fig 2] la figure 2 est un schéma fonctionnel illustrant les interactions entre les différents modules du système de la figure 1 ;
- [Fig 3] la figure 3 est un logigramme illustrant un premier exemple de mise en œuvre d'un procédé de détermination d'état opérationnel d'un équipage par l'intermédiaire du système de la figure 1 ;
- [Fig 4] la figure 4 est un logigramme illustrant un deuxième exemple de mise en œuvre d'un procédé de détermination d'état opérationnel d'un équipage par l'intermédiaire du système de la figure 1 ;
- [Fig 5] la figure 5 est un logigramme illustrant un troisième exemple de mise en œuvre d'un procédé de détermination d'état opérationnel d'un équipage par l'intermédiaire du système de la figure 1 ; et
- [Fig 6] la figure 6 est un logigramme illustrant un quatrième exemple de mise en œuvre d'un procédé de détermination d'état opérationnel d'un équipage par l'intermédiaire du système de la figure 1.
- [Fig 7] la figure 7 est un logigramme illustrant un cinquième exemple de mise en œuvre d'un procédé de détermination d'état opérationnel d'un équipage par l'intermédiaire du système de la figure 1.

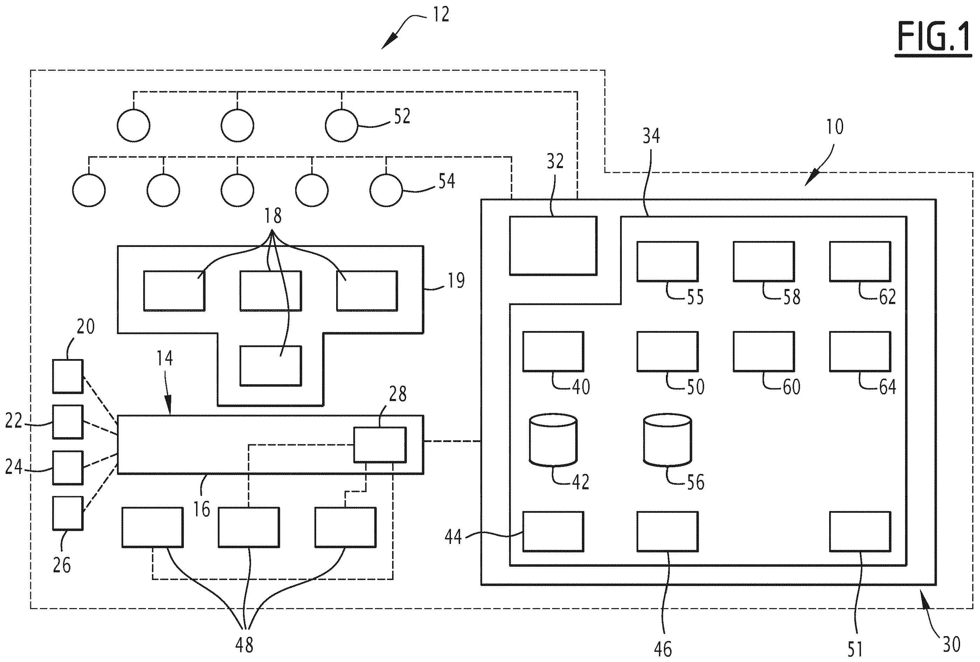

Un premier système 10 de détermination d'état opérationnel d'équipage d'un aéronef 12, en fonction d'un plan de tâches à réaliser par l'équipage de l'aéronef 12, est illustré schématiquement sur la figure 1.

Le système 10 est destiné à être relié à ou à être intégré dans un système central d'avionique 14 comportant une unité centrale d'avionique 16 et au moins une unité d'affichage 18 placée dans une interface de contrôle 19 de l'aéronef 12.

L'interface de contrôle 19 de l'aéronef 12 est par exemple situé dans l'aéronef 12 lui-même (dans le cockpit), ou dans une salle de commande à distance de l'aéronef 12 (dans une station sol).

L'unité centrale d'avionique 16 est notamment raccordée à des équipements de l'aéronef 12, destinés à interagir au sein de systèmes fonctionnels de l'aéronef.

Les systèmes fonctionnels de l'aéronef 12 incluent par exemple des systèmes 20 de mesure de l'état de l'aéronef, des systèmes 22 de communication extérieure, et des systèmes 24 d'actionnement des commandes de l'aéronef. Les systèmes fonctionnels de l'aéronef 12 comprennent en outre avantageusement des systèmes d'armes 26, dans le cas d'un avion militaire.

Les systèmes de mesure 20 comportent par exemple des composants comprenant des capteurs de mesure de paramètres extérieurs à l'aéronef, tels que la température, la pression ou la vitesse, des capteurs de mesure de paramètres internes à l'aéronef et à ses différents systèmes fonctionnels et des capteurs de positionnement, tels que des capteurs GPS, des centrales à inertie, et/ou un altimètre.

Les systèmes de communication extérieure 22 incluent par exemple des composants comprenant des systèmes radio, VOR/LOC, ADS, DME, ILS, des systèmes radars, et/ou des systèmes de communication par satellite tel que « SATCOM ».

Les systèmes de commande 24 incluent des composants comprenant des actionneurs propres à actionner des commandes de l'aéronef, tels que des volets, des gouvernes, des pompes, ou encore des circuits mécaniques, électriques ou/et hydrauliques, et des actionneurs logiciels propres à configurer les états avioniques de l'aéronef.

Les systèmes d'armes 26, lorsqu'ils sont présents, comprennent avantageusement des composants de repérage de cibles et de guidage d'armes et des armes en tant que telles, telles que des bombes ou des missiles.

Les différents systèmes 20 à 26 sont raccordés à l'unité centrale d'avionique 16, par exemple de manière numérique, par au moins un bus de données circulant sur un réseau interne à l'aéronef 12.

L'unité centrale d'avionique 16 comporte au moins un système 28 de gestion de tâches propre à établir, en temps réel, une liste de tâches équipage à effectuer, qui dépend d'objectifs de la mission actualisés par les mesures de l'environnement effectuées par exemple par les systèmes de mesure 20 ou reçues par les systèmes de communication 22.

Les tâches à effectuer sont par exemple des tâches de navigation, pour guider l'aéronef 12 et le déplacer suivant un plan de vol comprenant au moins des points géographiques de passage de l'aéronef 12 lors de la mission et des temps de passage aux points de passage.

Également, les tâches à effectuer sont par exemple des tâches de communication à effectuer suivant un plan de communication comportant des points géographiques de communication et/ou des temps de passage en des points géographiques de communication.

En outre, les tâches à effectuer peuvent être des tâches liées à des évènements extérieurs à l'aéronef, tels que la météorologie, comprenant des points géographiques de passage ou d'évitement de phénomènes météorologiques, par exemple, dans des zones de turbulence et/ou d'orage.

Dans le cadre d'un avion militaire, les tâches à effectuer comprennent en outre avantageusement des tâches de reconnaissance, des tâches de préparation à un combat aérien ou air-sol, et des tâches de combat aérien ou air-sol tel incluant par exemple de l'analyse d'image ou un tir d'arme effectué depuis l'aéronef.

La liste des tâches est généralement déterminée initialement, avant le vol à partir d'un système de préparation de mission, à partir d'objectifs de la mission. Les tâches sont mises à jour en fonction de l'exécution de la mission, par exemple parce que des nouveaux objectifs de mission sont déterminés par le commandant de la mission en fonction des pannes et/ou des urgences et/ou des évènements extérieurs météorologiques ou tactiques.

Le système de gestion de tâches 28 est alors propre à modifier la liste des tâches, pour ajouter, supprimer, ou/et changer l'ordre ou les caractéristiques des tâches à effectuer par l'équipage, en fonction d'objectifs de mission actualisés et d'un contexte de mission actualisé en fonction l'environnement autour de l'aéronef 12. Le plan de tâches comprenant la liste des tâches est ainsi adaptatif. Ces modifications sont réalisées dans la limite de délégation du système prévue par l'équipage et peuvent être soumise à accord de l'équipage.

Le système de détermination 10 d'état opérationnel d'équipage est illustré schématiquement sur la figure 1. Dans cet exemple, le système 10 comporte un calculateur 30 comprenant un processeur 32 et une mémoire 34 recevant des modules logiciels propres à être exécutés par le processeur 32 pour réaliser des fonctions. En variante, le calculateur 30 comporte des composants logiques programmables ou des circuits intégrés dédiés, destinés à réaliser les fonctions des modules qui seront décrits ci-après.

En référence à la figure 1, la mémoire 34 contient un module 40 de détermination de tâches d'équipage à effectuer à l'instant courant, propre à récupérer une liste de tâches à effectuer par l'équipage, en fonction d'objectifs de mission actualisés et/ou du contexte de mission actualisé, et à attribuer les tâches aux membres d'équipage, en fonction de données de profils équipage contenus dans une base de données 42.

La mémoire 34 contient en outre un module 44 de détermination de ressources équipage, propre à calculer les ressources nécessaires à chaque membre d'équipage pour effectuer chaque tâche de la liste de tâches qui lui est affectée et à déterminer un état opérationnel théorique de référence de chaque membre d'équipage, pour effectuer les tâches lui étant affectées, sur la base d'un modèle de ressources.

La mémoire 34 contient également un module 46 de détermination d'au moins un indicateur de contexte, propre à définir au moins un indicateur de contexte sur la base de données de contexte de la mission élaborées à partir de capteurs 48 des systèmes de mesures 20 de l'aéronef 12, ou de données transmises à l'aéronef 12 via les systèmes de communication 20.

La mémoire 34 contient aussi un module 51 de détermination des interactions, propre à déterminer les interactions attendues avec les systèmes avion, lors de la mise en œuvre des tâches de la liste de tâches par chaque membre d'équipage.

La mémoire 34 contient en outre un module 50 de mesure d'états d'interactions avec les systèmes avion, sur la base de mesures effectuées par des capteurs de mesures d'interaction 52 et des interactions attendues déterminées par le module 51 et un module 55 de mesure d'états physiologiques de l'équipage raccordé à des capteurs 54 de mesures de données physiologiques de l'équipage de l'aéronef 12 et à une base de données 56 de données physiologiques basiques de l'équipage.

La mémoire 34 contient enfin un module 58 de détermination d'états cognitifs locaux, propre à évaluer au moins un état cognitif local de l'équipage vis à vis de l'exécution de chaque tâche définie dans la liste des tâches à effectuer, et un module 60 de détermination d'états cognitifs globaux de l'équipage liés à l'état physiologique général de l'équipage vis-à-vis de l'ensemble des tâches à réaliser.

Un état cognitif local de l'équipage est un état d'un processus mental d'un membre d'équipage associé à la réalisation d'une tâche particulière à effectuer au sein de la liste des tâches.

Un état cognitif global de l'équipage est un état d'un processus mental d'un membre d'équipage associé à l'ensemble des tâches à effectuer par le membre d'équipage.

Selon l'invention, la mémoire 34 contient également un module 62 de détermination d'état opérationnel de l'équipage, propre à comparer au moins un état opérationnel effectif d'un membre d'équipage, en particulier, un état cognitif global et/ou un état cognitif local déterminés par les modules 58, 60, avec un état opérationnel théorique de référence, en particulier avec un état cognitif global théorique de référence et/ou un état cognitif local théorique de référence, déterminés par le module de détermination 44 à l'aide du modèle de ressources.

La mémoire 34 contient en outre un module 64 de reconfiguration de tâches sur la base de l'état opérationnel obtenu par le module de détermination d'état 62, propre à propre à modifier la liste de tâches à effectuer par le membre d'équipage ou les informations fournies à l'équipage.

Le module de détermination de tâches 40 est propre à importer, à chaque instant, les tâches provenant du système de gestion de tâches 28 pour obtenir une liste ordonnée de tâches, incluant par exemple l'identification de cibles, le suivi du plan de vol, les tâches de tirs à effectuer, etc ...

Chaque tâche est associée à au moins un membre d'équipage, dont le profil est obtenu dans la base de données 42. Le profil d'équipage comprend, par exemple, un niveau d'expertise du membre d'équipage à effectuer les tâches de la liste de tâches, ainsi que des modes d'interaction privilégiés.

Les modes d'interaction privilégiés sont par exemple des stratégies alternatives possibles dans la réalisation des tâches, en particulier des variantes d'ordres d'enchainement des tâches. Par exemple, certaines stratégies de réalisation d'une tâche sont connues d'un utilisateur expert ayant une expertise dans la réalisation de la tâche, lui permettant par exemple d'utiliser des raccourcis ou d'optimiser ses interactions avec les interfaces.

Le module 44 de détermination de ressources est propre, sur la base des listes de tâches, et sur la base des ressources nécessaires pour mettre en œuvre la liste de tâches, obtenues à partir d'un modèle de ressources, à déterminer, pour chaque membre d'équipage, au moins un état opérationnel théorique de référence du membre d'équipage, notamment au moins un état cognitif global théorique de référence ou/et au moins un état cognitif local théorique de référence associé à chaque tâche pour le membre d'équipage.

Des exemples d'états cognitif globaux de référence sont un niveau théorique de charge mentale accepté pour le membre d'équipage, un niveau théorique de somnolence accepté pour le membre d'équipage, un niveau théorique d'engagement du membre d'équipage accepté pour le membre d'équipage, un niveau théorique d'hypoxie accepté pour le membre d'équipage (ce niveau accepté étant un niveau nul) et/ou un niveau théorique de stress accepté pour le membre d'équipage.

Le niveau de charge mentale est un indicateur numérique représentatif de la demande d'utilisation des capacités mentales du membre d'équipage. Le niveau de somnolence est un indicateur numérique représentatif du degré d'ensommeillement du membre d'équipage, notamment dans un état intermédiaire entre la veille et le sommeil. Le niveau d'engagement est un indicateur numérique traduisant l'attention globale du membre d'équipage pour la réalisation des tâches. Le niveau d'hypoxie est un indicateur booléen représentatif de l'adéquation ou de l'inadéquation entre les besoins tissulaires en oxygène et les apports en oxygène pour le membre d'équipage. Le niveau de stress est un indicateur numérique représentatif de la tension nerveuse du membre d'équipage.

Ces niveaux théoriques sont prédéfinis globalement en fonction du profil équipage et sont destinés à être comparés à des niveaux réels, déterminés à partir de données mesurées sur l'équipage, comme décrit plus bas. Ces niveaux sont par exemple des niveaux numériques mesurant une valeur dans un intervalle de valeurs possibles, des niveaux discrets mesurant une valeur parmi une pluralité de valeurs discrètes possibles, ou des niveaux booléens présentant soit une valeur acceptable, soit une valeur non acceptable.

Des exemples d'états cognitifs locaux de référence pour la réalisation d'une tâche sont un niveau d'attention minimum, un niveau de persévération dans la réalisation de chaque tâche, et un niveau de tunnélisation visuelle ou/et auditive maximum pour effectuer chaque tâche de la liste des tâches.

Le niveau d'attention associé à une tâche est un indicateur numérique qui traduit la disponibilité du membre d'équipage à réaliser la tâche, en particulier la capacité d'un équipage à sélectionner et à se concentrer sur des informations spécifiques d'une tâche. Le niveau de persévération est un indicateur numérique qui traduit dans un contexte qui le nécessite un manque de révision d'une décision préalablement prise dans la réalisation d'une tâche. Le niveau de tunnélisation est un indicateur numérique traduisant l'allocation de l'attention de l'équipage à un canal sensoriel spécifique, au détriment des autres, ce qui entraine une inaptitude de l'équipage à prendre en compte certains types de stimuli (par exemple sonore et/ou visuel) liés à la tâche à réaliser.

Ces niveaux sont définis en fonction du profil équipage du membre d'équipage et de la liste des tâches affectée au membre d'équipage.

Les états cognitifs théoriques de référence sont déterminés à partir du modèle de ressources qui relie avantageusement chaque tâche à effectuer à un temps et/ou à un niveau de ressource à mettre en œuvre par le membre d'équipage pour effectuer la tâche.

Le module de détermination 44 est ainsi propre à affecter pour l'ensemble des tâches le temps nécessaire ainsi que l'état global nécessaire (par exemple : la charge mentale nécessaire) pour effectuer les tâches en fonction du profil du membre d'équipage donné par la base de données 42 et des indicateurs de contexte définis par le module de détermination d'indicateurs de contexte 46. De même, il est propre à affecter pour chaque tâche de la liste des tâches le temps nécessaire ainsi que l'état local nécessaire (par exemple : le niveau d'attention nécessaire) pour effectuer ladite tâche en fonction du profil du membre d'équipage.

Le module de détermination d'indicateurs de contexte 46 est propre à définir un niveau de complexité et/ou un niveau de danger, en fonction de mesures obtenues par les capteurs d'environnement 48, en particulier sur le nombre d'adversaires présents dans l'environnement de la mission, de l'état de la météorologie, et/ou de la présence des zones dangereuses.

Le niveau de complexité et/ou le niveau de danger est utilisé par le module de détermination 44 pour pondérer les ressources nécessaires pour exécuter chaque tâche, en particulier en termes de temps nécessaire ou de charge mentale nécessaire pour effectuer les tâches. Par exemple, si le niveau de complexité ou le niveau de danger augmente, le temps et la charge mentale nécessaires pour effectuer une tâche sont susceptibles d'augmenter.

Le module 51 de détermination des interactions est propre à déterminer les interactions attendues avec les systèmes avion par chaque membre d'équipage, et la performance attendue pour réaliser ces interactions, sur la base de la liste des tâches affectées au membre d'équipage obtenue à partir du module de détermination 40, du profil d'équipage du membre d'équipage, tel que déterminé dans la base de données 42 et des indicateurs de contexte déterminés par le module de détermination 46.

Les interactions avec les systèmes avion sont par exemple des zones d'écran ou des éléments à observer, des touches ou des régions d'écran à sélectionner, ou encore des commandes à actionner. La performance attendue est par exemple une vitesse minimum d'enchainement des actions à effectuer pour mettre en œuvre une ou plusieurs tâches, un taux d'erreur dans la mise en œuvre des tâches ou encore un taux de réalisation total de tâches parmi toutes les tâches à réaliser.

Les capteurs 52 de mesures d'interactions sont par exemple aptes à déterminer la position du regard sur les unités d'affichage 18 du cockpit 19, l'appui et la force d'appui sur les commandes, la position d'un curseur de commande des systèmes avion sur une unité d'affichage 18 ou sur une autre interface.

Sur la base des mesures effectuées par les capteurs de mesures d'interactions 52, le module de mesure d'états d'interactions 50 est propre à comparer les interactions avec les systèmes avion effectuées par le membre d'équipage et les interactions avec les systèmes avion attendues pour le membre d'équipage obtenues à partir du module 51 sur la base de la liste ordonnée de tâches, pour déterminer si le membre d'équipage est en train d'effectuer chacune des interactions attendues.

Le module de mesure d'états d'interactions 50 est en outre propre à déterminer si le membre d'équipage répond à la performance attendue en termes de rapidité d'exécution, de taux d'erreurs et de taux de réalisation.

Sur la base des performances et des actions exécutées par le membre d'équipage, le module de mesure d'états d'interactions 50 est propre à déterminer un niveau d'attention du membre d'équipage pour effectuer chaque tâche, un niveau de performance du membre d'équipage dans l'exécution de chaque tâche et un niveau d'efficacité de la stratégie employée pour gérer les tâches en fonction des modes d'interactions privilégiés définis plus haut.

Les capteurs de mesures physiologiques 54 comprennent par exemple des capteurs de mesure de la fréquence cardiaque, du diamètre pupillaire, de l'oxygénation du sang, des ondes cérébrales émises, de la posture (en particulier via des nappes de pression dans le siège ou/et via des systèmes d'analyse d'image par caméras), de la sudation, ou de l'oxygénation frontale.

Les capteurs de mesures physiologiques 54 peuvent être partiellement communs avec les capteurs de mesures d'interactions 52.

La base de données physiologiques 56, comporte, pour chaque membre d'équipage, des données d'états physiologiques basiques de chaque membre d'équipage au repos.

Ces données d'états physiologiques basiques sont par exemple une fréquence cardiaque au repos, un diamètre pupillaire au repos, un taux d'oxygénation du sang au repos, un taux d'ondes cérébrales émises au repos, une posture au repos, un taux de sudation au repos, un taux d'oxygénation frontale au repos.

Sur la base des mesures effectuées par les capteurs de mesures physiologiques 54, comparées aux données d'états physiologiques basiques présentes dans la base de données 56, le module de mesure d'état physiologiques 55 est propre à déterminer notamment un niveau de fatigue mentale du membre d'équipage, et/ou un niveau de concentration du membre d'équipage.

Le niveau de fatigue mentale est par exemple déterminé à partir d'un pourcentage mesuré de fermeture des yeux, d'un nombre et d'une durée des clignements, d'un niveau de relâchement de la posture, d'un niveau de répétition de gestes, et/ou de certaines puissances spectrales fréquentielles (par exemple ondes alpha) prédominantes dans le cerveau.

Le niveau de concentration est par exemple déterminé à partir d'un niveau d'oxygénation préfrontale, par exemple d'un niveau élevé d'oxygénation préfrontale, d'une variabilité de la fréquence cardiaque, par exemple d'une faible variabilité de la fréquence cardiaque, d'une variation de dispersion oculaire, par exemple d'une diminution de dispersion oculaire, et de certaines puissances spectrales fréquentielles du cerveau élevées (ondes theta dans la zone frontale)

Le module de détermination d'états cognitifs locaux 58 est propre à utiliser les données produites par le module de mesure d'états physiologiques 55 et le module de mesure d'états d'interactions 50 pour déterminer des états cognitifs locaux, liés à la réalisation de chacune des tâches spécifiques qui sont prévues dans la liste ordonnée de tâches.

Comme indiqué plus haut, les états cognitifs locaux comprennent par exemple un niveau d'attention dans la réalisation des tâches, par exemple déterminé à partir de l'analyse de la répartition du regard du pilote sur ses interfaces homme/machine.

Les états cognitifs locaux comprennent par exemple un niveau de persévération. Le niveau de persévération est déterminé par exemple à partir de mesures fréquentielles du cerveau, par un taux de réaction aux stimuli, en particulier un taux faible de réaction aux stimuli, par un taux de fixation sur la tâche, en particulier un taux élevé de fixation sur la tâche.

Un niveau de persévération élevé est par exemple obtenu lorsqu'un pilote continue à tout prix à tenter de se poser alors que les conditions externes imposeraient une remise des gaz.

Les états cognitifs locaux comprennent également de préférence un niveau de tunnélisation visuelle ou/et auditive, qui traduit une trop forte attention envers une tâche précise, en négligeant les autres tâches de la liste de tâches ou/et les stimuli visuels ou/et auditifs externes à la tâche. Le niveau de tunnélisation est déterminé par exemple à partir de mesures oculaires, de détermination d'absence d'interaction sur certaines tâches, et de détermination d'absence de réponse à certains stimuli (traduits par exemple par l'amplitude des potentiels évoqués P300 dans le cerveau, en particulier par une amplitude faible).

Le module de détermination d'états cognitifs globaux 60 est propre à utiliser les données produites par le module de mesure d'états physiologiques 55 et le module de mesure d'états d'interactions 50 pour déterminer des états cognitifs globaux de chaque membre d'équipage.

Les états cognitifs globaux comprennent par exemple un niveau de charge mentale global. Ce niveau de charge mentale globale est déterminé par exemple à partir de l'oxygénation préfrontale, en particulier d'une forte oxygénation préfrontale, de la variabilité du rythme cardiaque, en particulier, d'une baisse de la variabilité du rythme cardiaque, de la performance sur les tâches secondaires (retard, etc), en particulier d'une baisse de performance, de la variation du diamètre pupillaire, en particulier, d'une augmentation du diamètre pupillaire.

Les états cognitifs globaux comprennent avantageusement un niveau de somnolence global. Ce niveau de somnolence globale est déterminé par exemple à partir de la posture, en particulier d'une posture relâchée, du nombre de clignements des yeux en particulier d'une augmentation du nombre de clignements des yeux, et d'une détermination d'absence d'interactions.

Les états cognitifs globaux comprennent avantageusement un niveau d'engagement, qui traduit le taux de tâches réalisées par rapport aux tâches à réaliser. Le niveau d'engagement est déterminé par exemple à partir d'un rapport des ondes fréquentielles du cerveau (beta/alpha+delta), nommé également "index d'engagement", notamment plus élevé qu'au repos, et/ou d'une analyse des données oculaires par exemple pour identifier un ratio "longues fixations + courtes saccades / longues saccades + courtes fixations", en particulier lorsque ce ratio est faible.

Les états cognitifs globaux comprennent avantageusement un niveau d'hypoxie, représentatif de l'inadéquation entre les besoins tissulaires en oxygène et les apports en oxygène pour le membre d'équipage. Ce niveau d'hypoxie est un niveau booléen. Il est déterminé par exemple à partir d'une fréquence cardiaque et respiratoire au-delà de seuils prédéterminés traduisant un anormalité, une baisse de la saturation en oxygène du sang en dessous d'un seuil, des phrases incohérentes, ou/et des clignements des yeux au-dessus d'un seuil déterminé.

Les états cognitifs globaux comprennent également de préférence un niveau de stress. Le niveau de stress est déterminé par exemple à partir d'une microsudation, en particulier d'une forte microsudation, d'une tension musculaire, en particulier d'une forte tension musculaire, d'une variabilité du rythme cardiaque, en particulier d'une forte variabilité du rythme cardiaque, d'un ratio saccades/fixations du regard, en particulier d'un fort ratio saccades/fixations, d'une fréquence de voix, en particulier d'une voix qui devient plus aigüe, ou/et d'une posture redressée sur le siège.

Le module de détermination d'état opérationnel équipage 62 est propre à analyser les états cognitifs globaux et les états cognitifs locaux et à les comparer respectivement aux états cognitifs globaux de référence et aux états cognitifs locaux de référence calculés par le module de détermination de ressources 44 pour déterminer un indicateur d'aptitude du membre d'équipage, par exemple booléen, propre à passer entre un état d'aptitude du membre d'équipage à effectuer les tâches de la liste des tâches qui lui ont été affectées et un état d'inaptitude du membre d'équipage à effectuer des tâches de la liste des tâches qui lui ont été affectées.

Par exemple, le module de détermination d'état opérationnel équipage 62 est propre à évaluer si le niveau de charge mentale effectif déterminé par le module de détermination d'états cognitifs globaux 60 est supérieur au niveau maximum de charge mentale théorique calculé par le module de détermination de ressources 44. Dans ce cas, il est propre à passer l'indicateur d'aptitude du membre d'équipage d'un état d'aptitude à un état d'inaptitude.

De même, le module de détermination d'état opérationnel équipage 62 est avantageusement propre à évaluer si le niveau de stress effectif déterminé par le module de détermination d'états cognitifs globaux 60 est supérieur au niveau maximum de stress calculé par le module de détermination de ressources 44. Dans ce cas, il est propre à passer l'indicateur d'aptitude du membre d'équipage d'un état d'aptitude à un état d'inaptitude.

Le module de détermination d'état opérationnel équipage 62 est avantageusement propre à évaluer si le niveau de somnolence effectif déterminé par le module de détermination d'états cognitifs globaux 60 est supérieur au niveau maximum de somnolence calculé par le module de détermination de ressources 44. Dans ce cas, il est propre à passer l'indicateur d'aptitude du membre d'équipage d'un état d'aptitude à un état d'inaptitude.

Le module de détermination d'état opérationnel équipage 62 est avantageusement propre à évaluer si le niveau d'engagement effectif déterminé par le module de détermination d'états cognitifs globaux 60 est supérieur au niveau d'engagement calculé par le module de détermination de ressources 44. Dans ce cas, il est propre à passer l'indicateur d'aptitude du membre d'équipage d'un état d'aptitude à un état d'inaptitude.

Le module de détermination d'état opérationnel équipage 62 est avantageusement propre à évaluer si le niveau d'hypoxie effectif déterminé par le module de détermination d'états cognitifs globaux 60 est supérieur au niveau d'hypoxie autorisé, ici un niveau nul. Dans ce cas, il est propre à passer l'indicateur d'aptitude du membre d'équipage d'un état d'aptitude à un état d'inaptitude.

En outre, le module de détermination d'état opérationnel équipage 62 est avantageusement propre à évaluer si le niveau de tunnélisation effectif ou/et le niveau de persévérance déterminés par le module de détermination d'états cognitifs locaux 58 dépassent respectivement le niveau de tunnélisation maximum théorique ou/et le niveau de persévérance maximum théorique déterminés par le module de détermination de ressources 44, et si le niveau d'attention déterminé par le module de détermination d'états cognitifs locaux 58 est inférieur au niveau d'attention déterminé par le module de détermination de ressources 44.

Dans chacun de ces cas, il est propre à passer l'indicateur d'aptitude du membre d'équipage d'un état d'aptitude à un état d'inaptitude.

Le module de détermination d'état opérationnel 62 est propre, lorsqu'un état d'inaptitude est déterminé, à définir un type de reconfiguration de tâches ou de modification d'information équipage à effectuer par le module de configuration de tâches 64.

Le module de configuration de tâches 64 est avantageusement propre à modifier la liste des tâches pour effectuer une tâche à la place de l'équipage, pour supprimer une tâche, pour reporter dans le temps une tâche à effectuer.

Il est propre également à engendrer une alerte et/ou une alarme dans le cas où le module de détermination d'état opérationnel 62 détermine un état d'inaptitude de l'équipage, ou encore à supprimer temporairement des informations affichées à l'équipage sur les unités d'affichage 18, ou/et à afficher de nouvelles informations remplaçant celles existantes.

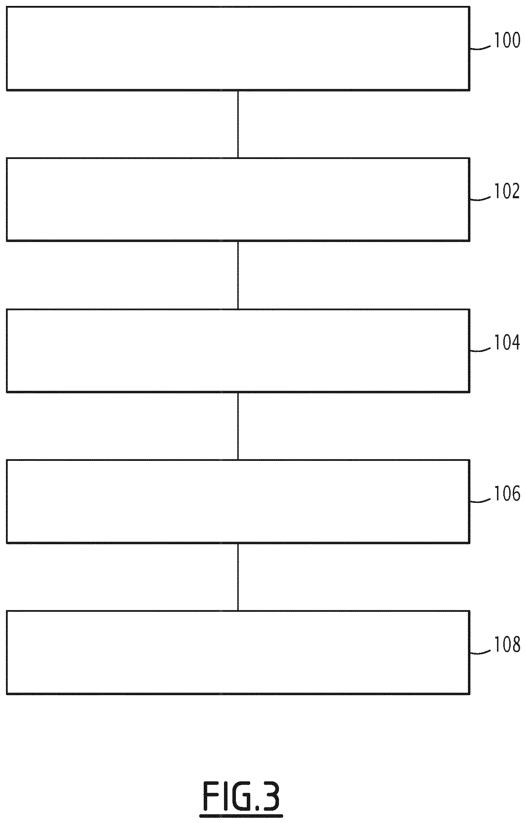

Des exemples de mise en œuvre d'un procédé de détermination de l'état opérationnel d'un équipage en fonction d'un plan de tâches à l'aide du système de détermination 10 selon l'invention vont maintenant être décrits, en regard des figures 3 à 7.

Dans l'exemple illustré par la figure 3, dans un contexte d'avion civil, l'aéronef 12 opère une phase de croisière, après le décollage de l'aéronef 12.

Le système de gestion de tâches 28 définit les tâches à effectuer, qui sont essentiellement des tâches de suivi de la trajectoire de l'aéronef, en particulier du passage par des points de passage, de la consommation en carburant de l'aéronef 12 et des tâches de communication, lors du passage dans divers espaces aériens.

Le membre d'équipage chargé du suivi est susceptible d'entrer dans un état de somnolence.

Comme indiqué plus haut, à l'étape 100 du procédé selon l'invention, le module de détermination de tâches 40 établit à chaque instant la liste des tâches à effectuer, et la répartition des tâches entre les membres d'équipage destinés à les effectuer en utilisant la base de données de profils équipage 42.

Puis, à l'étape 102, le module de détermination de ressources 44 définit un niveau maximum de somnolence accepté pour effectuer ces tâches, sur la base par exemple d'un niveau de somnolence maximum associé à chaque tâche individuelle.

A l'étape 104, sur la base des mesures obtenues à partir des capteurs physiologiques 54, le module de mesure d'états physiologiques 55 détermine une augmentation de la fréquence et de la durée des clignements des yeux, une augmentation du pourcentage de fermeture des paupières, et une posture relâchée.

Sur la base de ces paramètres, le module de détermination des états cognitifs globaux 60 calcule un niveau de somnolence effectif.

A l'étape 106, le module de détermination d'état opérationnel 62 détermine que le niveau de somnolence effectif est supérieur au niveau maximum de somnolence admissible pour l'ensemble des tâches en cours. Il passe donc l'indicateur d'aptitude du membre d'équipage, de l'état d'aptitude du membre d'équipage à effectuer les tâches de la liste des tâches qui lui ont été affectées à l'état d'inaptitude du membre d'équipage à effectuer des tâches de la liste des tâches qui lui ont été affectées.

Le module de détermination d'état opérationnel 62 détermine également que le type de reconfiguration est de déclencher une alarme de réveil et d'automatiser temporairement le pilotage de l'aéronef.

A l'étape 108, le module de reconfiguration 64 envoie donc des instructions au système avion pour engendrer une alarme dans le cockpit 19.

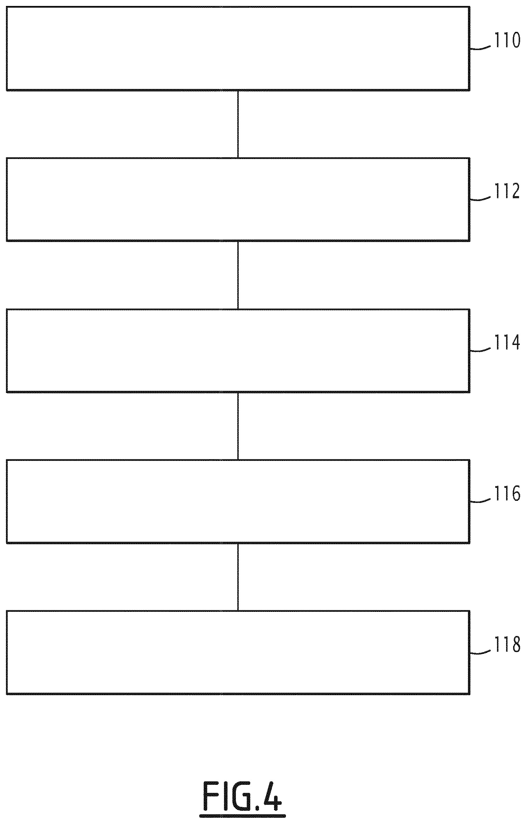

Dans un deuxième exemple, illustré par la figure 4, lors d'une phase d'approche vers une piste d'atterrissage avec un fort vent de travers, le pilote est concentré sur une tâche de pilotage.

Si une alarme importante se déclenche, il est susceptible de ne pas la traiter de manière prioritaire.

A cet égard, le module de détermination de tâches 40 détermine une liste des tâches relatives au pilotage, et une liste de tâches relative au traitement de l'alarme, qui peut être une alarme CAS rouge requérant une action prioritaire du pilote à partir de la liste des tâches.

A l'étape 110, le module 51 de modélisation des interactions détermine que le membre d'équipage devrait traiter les tâches associées à l'alarme, en effectuant un certain nombre d'interactions dans le cockpit 19 notamment sur les unités d'affichage 18 et/ou à l'aide des commandes.

A l'étape 112, le module de mesure d'états d'interactions 50 détermine, à partir de la position du curseur, de la position du regard, que le membre d'équipage n'effectue aucune des interactions liées à l'alarme.

En outre, le module de mesure d'états d'interactions 50 établit que le membre d'équipage ne réagit pas aux stimuli sonores et visuels résultant de l'alarme qui sont en dehors de son champ de vision.

Il détermine sur cette base un niveau d'attention du membre d'équipage pour effectuer chaque tâche, et un niveau de performance du membre d'équipage dans l'exécution de chaque tâche.

De même, à l'étape 114, le module de mesure d'états physiologiques 55 détermine un diamètre pupillaire avec peu de dispersion visuelle, une variabilité de fréquence cardiaque faible, par exemple inférieure à 20% par rapport à la variabilité au repos et une fréquence cardiaque élevée par rapport à la fréquence cardiaque au repos, par exemple au moins supérieure à 20% de la fréquence cardiaque au repos, à partir des bases de données 56 de données physiologiques de base.

Le module de mesure d'états physiologiques 55 détermine en outre une absence d'amplitude des potentiels évoqués P300 suite à un stimulus sonore (alarme).

Sur la base des informations obtenues par les modules 50, 55, le module de détermination d'états cognitifs globaux 60 détermine un niveau de charge mentale, et un niveau de stress. Le module de détermination d'états cognitifs locaux 58 détermine un niveau de tunnélisation.

A l'étape 116, le module de détermination d'état opérationnel 62 détermine alors que le niveau de charge mentale effectif est supérieur au niveau maximum de charge mentale déterminé par le module de détermination 44 et que le niveau de tunnélisation dépasse le niveau maximum de tunnélisation déterminé par le module de détermination 44. Il définit un état d'inaptitude du membre d'équipage à réaliser les tâches demandées, en particulier à traiter les alarmes de mission. Le membre d'équipage est ici dans un état de surdité attentionnelle.

Le module de détermination d'état opérationnel 62 détermine en outre que l'action à effectuer serait de supprimer temporairement l'affichage des informations sur lesquelles le pilote se concentre.

A l'étape 118, le module de reconfiguration 64 pilote donc les unités d'affichage 18 dans le cockpit 19 pour supprimer au moins une information relative au pilotage par vent de travers, afin de permettre au pilote d'identifier qu'une autre alarme urgente est présente et doit être traitée.

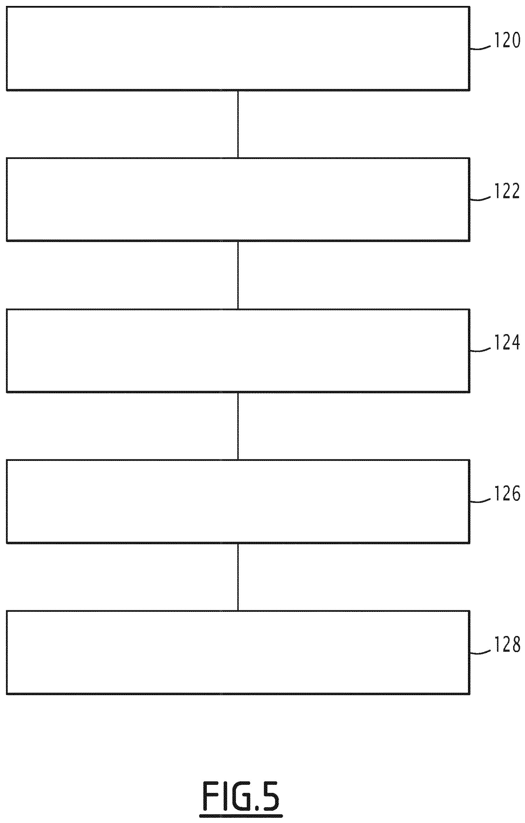

Dans un troisième exemple, représenté sur la figure 5, le système 10 détecte un état de panique présent chez un membre d'équipage.

Ceci peut se produire par exemple lors d'un affichage multiple d'alarmes.

Comme précédemment, à l'étape 120, le module de détermination 40 de tâches à effectuer détermine qu'une pluralité de tâches doivent être effectuées simultanément et définit pour chaque tâche un niveau de charge mentale et un niveau de stress associé à la tâche.

Le module 51 de modélisation des interactions détermine les interactions attendues avec l'interface du cockpit et les performances attendues pour réaliser les tâches de suivi.

A l'étape 122, sur la base des mesures des capteurs de mesures d'interactions 52, le module de mesure d'états d'interactions 50 compare les interactions attendues pour chaque membre d'équipage avec les systèmes avion obtenues à partir du module 51 sur la base de la liste ordonnée de tâches. Il identifie une dispersion dans les interactions, et le fait qu'aucune tâche n'est menée à son terme.

Il calcule donc un niveau d'attention et un niveau d'engagement. Ces niveaux sont faibles.

Par ailleurs, à l'étape 124, sur la base des capteurs physiologiques 54, le module de mesure d'états physiologiques 55 détermine la présence de nombreuses saccades dans le déplacement des yeux, une fréquence cardiaque très élevée, et une sudation soudaine et forte.

Sur cette base, le module de détermination d'états cognitifs globaux 60 calcule un niveau de stress. Ce niveau de stress est élevé.

A l'étape 126, le module de détermination d'état opérationnel 62 identifie alors un niveau de stress élevé, supérieur au niveau maximum de stress défini par le module de détermination 44. Ce niveau de stress est caractéristique d'un état de panique qui rend le membre de l'équipage inapte à réaliser les tâches critiques pour la sécurité.

Il identifie un niveau d'attention et un niveau d'engagement inférieurs respectivement aux niveau minimum d'attention et au niveau minimum d'engagement déterminés par le module de détermination 44.

Il définit donc un état d'inaptitude du membre d'équipage à réaliser les tâches demandées

Le module de détermination d'état opérationnel 62 détermine en outre qu'une synthèse des informations et de la procédure à suivre doit être effectuée par le module de reconfiguration 64.

A l'étape 128, le module de reconfiguration pilote les unités d'affichage 18 de l'unité centrale d'avionique 16 pour dispenser une synthèse orale et graphique des informations et de la procédure à suivre.

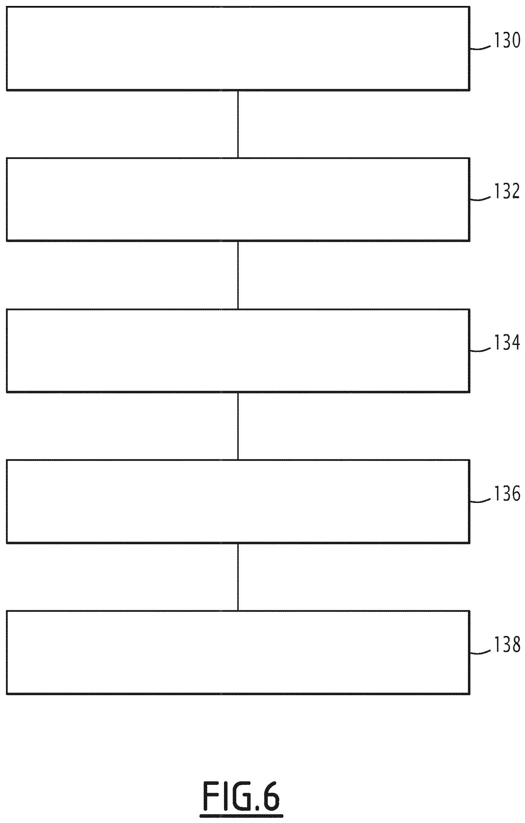

Un autre exemple va maintenant être décrit en référence à la figure 6, dans le contexte d'un avion militaire lors d'un combat aérien en haute altitude.

Comme précédemment, à l'étape 130, le module de détermination de tâches 40 détermine la liste des tâches à effectuer lors de cette phase de combat aérien. Le module 51 de détermination des interactions détermine les interactions attendues avec les systèmes avion par le membre d'équipage, et la performance attendue pour réaliser ces interactions, sur la base de la liste des tâches affectées au membre d'équipage obtenue à partir du module de détermination 40, du profil d'équipage du membre d'équipage, tel que déterminé dans la base de données 42 et des indicateurs de contexte déterminés par le module de détermination 46.

A l'étape 132, sur la base des données des capteurs d'interaction 52, le module de mesure des états d'interaction détermine que le temps de réaction augmente significativement, et que les réponses ne correspondent pas à ce qui est attendu au niveau des interactions.

Le module de détermination des états cognitifs globaux 60 détermine ainsi un niveau d'engagement, et le module de détermination des états cognitifs locaux 58 détermine un niveau d'attention. Ces niveaux sont faibles.

En parallèle, à l'étape 134, sur la base des données des capteurs physiologiques 54, le module de mesure d'états physiologiques 55 détermine une diminution de la saturation sanguine en oxygène, et une posture anormale caractérisée par une absence de mouvements.

Le module de détermination des états cognitifs globaux 60 en déduit un niveau d'hypoxie. Ce niveau d'hypoxie est inacceptable.

A l'étape 136, le module de détermination d'état opérationnel 62 identifie que le niveau d'hypoxie est supérieur à un niveau maximal d'hypoxie déterminé par le module de détermination 44, ici un niveau nul. Il définit donc un état d'inaptitude du membre d'équipage à réaliser les tâches demandées.

Il détermine également les actions à effectuer pour le module de reconfiguration 64, qui consistent à laisser l'unité centrale d'avionique 16 reprendre le pilotage, et à effectuer une descente d'urgence.

A l'étape 138, le module de reconfiguration 64 envoie des ordres à l'unité centrale d'avionique 16 pour effectuer cette reprise en main et la descente d'urgence.

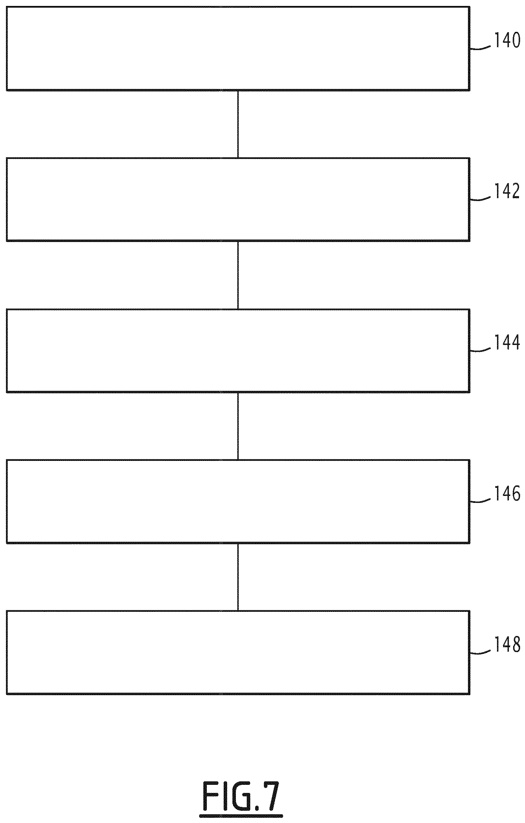

Dans un autre exemple, représenté sur la figure 7, toujours dans le contexte d'un avion militaire, lors d'une mission de combat air-sol, le pilote est contraint à réaliser de nombreuses tâches en un temps limité.

A l'étape 140, le module de détermination de tâches 40 détermine la liste des tâches à effectuer. Le module 51 de détermination des interactions détermine les interactions attendues avec les systèmes avion par le membre d'équipage, et la performance attendue pour réaliser ces interactions, sur la base de la liste des tâches affectées au membre d'équipage obtenue à partir du module de détermination 40, du profil d'équipage du membre d'équipage, tel que déterminé dans la base de données 42 et des indicateurs de contexte déterminés par le module de détermination 46.

A l'étape 142, sur la base des données des capteurs d'interaction 52, le module de mesure des états d'interaction 50 détermine des erreurs répétitives sur les interactions à effectuer et une diminution des performances, notamment sur des tâches qui sont non prioritaires ou non prévues dans la liste des tâches initiales.

Le module de détermination des états cognitifs globaux 60 détermine ainsi un niveau d'engagement, et le module de détermination des états cognitifs locaux 58 détermine un niveau d'attention. Ces niveaux sont très élevés.

En parallèle, à l'étape 144, sur la base des données des capteurs physiologiques 54, le module de mesure d'états physiologiques 55 détermine une forte oxygénation préfrontale, supérieure à un seuil donné, la dominance d'ondes thêta et bêta au niveau frontal, un diamètre pupillaire important, une variabilité de fréquence cardiaque très faible. Il en déduit un niveau de concentration élevé,

Le module de détermination des états cognitifs globaux 60 calcule sur cette base un niveau de charge mentale. Ce niveau de charge mentale est élevé.

A l'étape 146, le module de détermination d'état opérationnel 62 détermine alors un niveau de charge mentale supérieur au niveau maximum de charge mentale déterminé par le module de détermination 44. Il définit donc un état d'inaptitude du membre d'équipage à réaliser l'ensemble des tâches demandées.

Le module de détermination d'état opérationnel 62 détermine alors un type de reconfiguration consistant à ce que l'unité centrale d'avionique 16 prenne en main automatiquement certaines tâches de priorité inférieure.

A l'étape 148, le module de reconfiguration 64 envoie donc des instructions à l'unité centrale d'avionique 16 pour qu'elle prenne en charge les tâches ainsi définies.

Le système de détermination 10 selon l'invention est donc apte à élaborer un état opérationnel de l'équipage, en fonction d'un modèle de ressources bâti sur la liste des tâches équipage, en mesurant de manière séparée, les états physiologiques et les états d'interaction de l'équipage, même si ces mesures proviennent à priori de données issues des mêmes capteurs et en déterminant de manière séparée des états cognitifs locaux, relatifs à l'exécution d'une tâche et des états cognitifs globaux, relatifs à l'ensemble des tâches.

Le système de détermination 10 actualise l'état opérationnel au fur et à mesure des modifications de la liste des tâches, par exemple dans le cas où une nouvelle tâche apparait, ou en fonction des modifications des objectifs de la mission, ou du contexte observé dans l'environnement.

Ceci rend le système de détermination 10 très interactif, puisqu'il prend en compte de manière dynamique le contexte de la mission, et les ressources de l'équipage.

Grâce à son une architecture fonctionnelle générique, le système de détermination 10 est en outre facilement paramétrable pour s'adapter à différents types de capteurs, de missions et de plateformes. Il tient compte des caractéristiques propres des membres d'équipage.

Avantageusement, le système de détermination 10 établit en outre des actions de reconfiguration aidant à résoudre les problèmes identifiés.

Ainsi, le système de détermination 10 interprète facilement l'état de l'équipage, ce qui améliore la performance de l'équipage, diminue son stress et sa charge mentale.

Comme énoncé plus haut, le module de détermination d'états cognitifs locaux 58 est propre à utiliser les données produites par le module de mesure d'états physiologiques 55 et le module de mesure d'états d'interactions 50 pour déterminer des états cognitifs locaux, liés à la réalisation de chacune des tâches spécifiques qui sont prévues dans la liste ordonnée de tâches.

De même, le module de détermination d'états cognitifs globaux 60 est propre à utiliser les données produites par le module de mesure d'états physiologiques 55 et le module de mesure d'états d'interactions 50 pour déterminer des états cognitifs globaux de chaque membre d'équipage.

Ainsi, un même capteur 54 de mesures physiologiques ou un même capteur de mesure d'interactions 52, peut être utilisé pour déterminer au moins un état cognitif local se référant à une tâche et au moins un état cognitif global.

Par exemple, lorsque le capteur 52, 54 est un oculomètre, l'observation des paramètres de pourcentage d'ouverture des yeux et de taux de clignement permettent avantageusement d'identifier le niveau de somnolence du membre d'équipage, qui est un état physiologique global. Par ailleurs, un pourcentage d'ouverture de yeux en forte baisse peut aussi indiquer un état de somnolence.

La zone de fixation du regard, associée au niveau de saccades oculaires, permet par exemple d'identifier un niveau d'attention, avantageusement défini par le taux de fixation attentionnelle sur une tâche donnée, sachant qu'une tâche peut être associée à un ensemble de zones d'attention dans le cockpit. Ceci caractérise un état physiologique local.

Dans le cas d'un capteur 52, 54 permettant d'établir électro-encéphalogramme, l'observation des ondes cérébrales établit des états physiologiques globaux (somnolence, charge mentale).

L'observation des réponses (potentiel électrique cérébral mesuré) à des stimuli auditifs au sein du cerveau (N100, P300) établit des états physiologiques locaux (par exemple un niveau d'attention porté sur une alarme ou d'une information).

Pour la détermination des états cognitifs effectifs, les modules de détermination 58, 60 sont propres à mettre en œuvre des algorithmes d'apprentissage automatique ou et des algorithmes sans apprentissage.

Les algorithmes d'apprentissage automatique peuvent être des réseaux de neurones ou/et des arbres de décision. Les algorithmes sans apprentissage sont par exemple des règles expertes.

L'entrainement des algorithmes est avantageusement effectué comme suit.

Les données de mesure des capteurs 52, 54 sont enregistrées sur des phases d'expérimentation effectuées par un nombre de membres d'équipage permettant un apprentissage supervisé.

L'entrainement comporte plusieurs étapes incluant une labélisation des données de mesure pour établir les états cognitifs locaux ou globaux.

Cette labélisation peut être réalisée manuellement par des observateurs extérieurs ayant assisté aux phases d'expérimentation, et validée par les membres d'équipages concernés ou bien manuellement, a posteriori, en revoyant un enregistrement vidéo de la phase d'expérimentation avec le membre d'équipage concerné.

En variante, la labélisation peut être réalisée automatiquement, en se basant sur les résultats issus de tests subjectifs posés aux membres d'équipage pendant les phases d'expérimentation (par exemple, test NASA-TLX pour la charge mentale) ou bien automatiquement, en se basant sur des résultats chiffrés de performance des membres d'équipage pendant l'enregistrement des données (score, temps de réaction...).

En variante, la labellisation est réalisée par un mélange de toutes ces techniques.

Les données labellisées sont ensuite rendues anonymes, normalisées par la ligne de base de chaque membre d'équipage afin de s'affranchir des variabilités interindividuelles, avantageusement mélangées et séparées en plusieurs ensembles permettant l'entraînement, la mise au point et le test des algorithmes.

Ensuite, des algorithmes d'apprentissage automatique sont entraînés à partir de ces données ou les règles métier des algorithmes sans apprentissage sont établies.

Dans un exemple, il est possible dans un premier temps de considérer une flotte d'avions existante et de faire des mesures de capteurs rendus anonymes sur tous les pilotes de la flotte, puis de corréler les mesures au sol après les vols avec l'état observé (suite à des questionnaires pilote, et/ou une labélisation d'experts), et ensuite d'intégrer ces résultats dans le système de détermination 10. Ceci peut être réalisé de façon itérative.

Ceci étant fait, lors d'un vol, le module de détermination 60 est apte à établir à chaque instant des états cognitifs globaux effectifs du membre d'équipage en fonction des mesures physiologiques et d'interaction du membre d'équipage et le module 58 de détermination est apte à établir à chaque instant des états cognitifs locaux effectifs relatifs à l'exécution de tâches par le membre d'équipage en fonction des mesures physiologiques et d'interaction du membre d'équipage, en mettant en œuvre les algorithmes précités.

De même, comme exposé plus haut, le module 44 de détermination de ressources équipage, est propre à déterminer pour le ou pour chaque membre d'équipage, au moins un état cognitif théorique de référence du membre d'équipage en fonction de la liste de tâches affectée au membre d'équipage, en utilisant un modèle de ressources.

Ces états cognitifs de référence sont avantageusement des états cognitifs globaux de référence (par exemple : niveau de charge mentale, niveau de somnolence) ou des états cognitifs locaux de référence (par exemple : priorisation de l'attention sur une tâche A par rapport à une tâche B).

Chaque état cognitif de référence global ou local déterminé à l'aide du modèle de ressources par le module 44 correspond à un état cognitif effectif global ou local déterminé par un module de détermination 58, 60.

Ainsi, durant le vol, le module de détermination 62 compare chaque état cognitif effectif global ou local déterminé par un module de détermination 58, 60 à un état cognitif de référence global ou local correspondant, déterminé à l'aide du modèle de ressources par le module 44, afin d'obtenir l'indicateur d'aptitude, comme décrit plus haut.

Le modèle de ressources est propre à déterminer au moins état cognitif global de référence (par exemple : la charge mentale nécessaire) en fonction de la ou des tâches courantes de la liste de tâches, en fonction du profil du membre d'équipage donné par la base de données 42, et éventuellement en fonction des indicateurs de contexte définis par le module de détermination d'indicateurs de contexte 46.

De même, le modèle de ressources est propre à déterminer au moins état cognitif local de référence (par exemple : le niveau d'attention nécessaire) en fonction d'au moins une tâche de la liste de tâches, en fonction du profil du membre d'équipage et éventuellement en fonction d'une priorisation définie des tâches.

Le modèle de ressources met avantageusement en œuvre des algorithmes d'apprentissage automatique ou et des algorithmes sans apprentissage.

Les algorithmes d'apprentissage automatique peuvent être des réseaux de neurones ou/et des arbres de décision. Les algorithmes sans apprentissage sont par exemple des règles expertes.

L'entrainement des algorithmes est avantageusement effectué sur des jeux de données créés ou par expertise.

L'entrainement des algorithmes est avantageusement effectué comme décrit plus haut, en mettant en œuvre des phases d'expérimentation.

Les états cognitifs de référence sont par exemple prédéfinis par expertise. En variante, des états cognitifs de référence sont labellisés à partir de missions qui se sont correctement déroulées, pour lesquelles l'opérateur a réagi en adéquation avec les tâches effectuées (par exemple avec un retour subjectif de l'opérateur comme quoi il n'a pas ressenti de problème sur les tâches effectuées).

Une fois le modèle de ressourcés entrainé ou réglé, il est possible de l'utiliser en lui fournissant en entrée une liste de tâches à effectuer, un profil du membre d'équipage donné par la base de données 42, et éventuellement des indicateurs de contexte définis par le module de détermination d'indicateurs de contexte 46.

Le modèle de ressources fournit alors en sortie au moins un état cognitif de référence, notamment un état cognitif global de référence correspondant à l'ensemble des tâches données en entrée (par exemple : niveau de charge mentale, niveau de somnolence) et/ou un état cognitif local de référence correspondant à au moins une tâche de la liste de tâches fournie en entrée (par exemple : priorisation de l'attention sur une tâche A par rapport à une tâche B).

Le module 62 de détermination d'état opérationnel d'équipage est alors propre à déterminer l'indicateur d'aptitude du ou de chaque membre d'équipage à effectuer la ou les tâches de la liste de tâches affectée au membre d'équipage, à partir de la comparaison entre chaque état cognitif effectif du membre d'équipage déterminé sur la base de mesures physiologiques sur le membre d'équipage et de mesures d'interaction du membre d'équipage avec l'aéronef 12, et l'état cognitif théorique de référence correspondant, déterminé par le modèle de ressources.

Dans un premier exemple illustratif, lors d'un transit pour un vol de longue durée, le modèle de ressources indique qu'un niveau de charge mentale élevée (supérieure à un seuil prédéfini) n'est pas normal, alors qu'un niveau de somnolence supérieur à un seuil prédéfini est acceptable.

Sur la base des mesures physiologiques et d'interaction, le module de détermination 60 établit que le pilote a une charge normale et est somnolent. Le module de détermination 62 établit alors que l'indicateur d'aptitude du membre d'équipage est dans l'état d'aptitude du membre d'équipage à effectuer les tâches de la liste des tâches.

Au contraire, lors d'un atterrissage avec une météorologie dégradée, le modèle de ressources indique qu'un niveau de charge mentale élevée (supérieure à un seuil prédéterminé) est acceptable, alors qu'un niveau de somnolence supérieur à un seuil prédéfini n'est pas acceptable.

Sur la base des mesures physiologiques et d'interaction, le module de détermination 60 établit que le pilote a une charge normale et est somnolent. Le module de détermination 62 établit alors que l'indicateur d'aptitude du membre d'équipage est dans l'état d'inaptitude du membre d'équipage à effectuer les tâches de la liste des tâches.

Dans un autre exemple illustratif, le modèle de ressources indique que le niveau de charge mentale du pilote (qui correspond à état global de référence) doit éviter de dépasser un niveau maximal prédéfini de référence, et que l'attention du pilote (qui correspond à un état local de référence) doit être portée sur une tâche A en priorité.

Sur la base des mesures physiologiques et d'interaction, le module de détermination 60 établit le niveau charge mentale du pilote, et le module de détermination 62 détermine que ce niveau effectif de charge mental est bien inférieur à la valeur du niveau maximal prédéfini de référence établi par le modèle de ressources.

Par contre, toujours sur la base des mesures physiologiques et d'interaction, le module de détermination 60 établit que l'attention du pilote est portée sur une tâche B moins prioritaire que la tâche A selon le modèle de ressources.

Le module de détermination 62 établit alors que l'indicateur d'aptitude du membre d'équipage est dans l'état d'inaptitude du membre d'équipage à effectuer les tâches de la liste des tâches.

## Revendications

1. Système (10) de détermination d'un état opérationnel d'un équipage d'aéronef (12) habité ou non habité, en fonction d'un plan de tâches adaptatif, le système (10) comportant :
- un module (40) de détermination de tâches d'équipage à effectuer, propre à définir, à chaque instant lors d'une mission de l'aéronef (12), une liste de tâches à effectuer par au moins un membre d'équipage en fonction d'objectifs de mission actualisés, et d'un contexte instantané de mission déterminé à partir de mesures d'environnement de la mission ; **caractérisé par** :
- un module (44) de détermination de ressources équipage, propre à déterminer pour le ou pour chaque membre d'équipage, au moins un état cognitif théorique de référence du membre d'équipage en fonction de la liste de tâches affectée au membre d'équipage, en utilisant un modèle de ressources ;
- un module (62) de détermination d'état opérationnel d'équipage, propre à déterminer un indicateur d'aptitude du ou de chaque membre d'équipage à effectuer la ou les tâches de la liste de tâches affectée au membre d'équipage, à partir d'un état cognitif effectif du membre d'équipage déterminé sur la base de mesures physiologiques sur le membre d'équipage et de mesures d'interaction du membre d'équipage avec l'aéronef (12), et à partir de l'état cognitif théorique de référence du membre d'équipage déterminé par le module de détermination de ressources équipage (44),
- un module (60) de détermination d'états cognitifs globaux du membre d'équipage en fonction des mesures physiologiques et d'interaction du membre d'équipage et
- un module (58) de détermination d'états cognitifs locaux de l'équipage, propre à déterminer des états cognitifs locaux relatifs à l'exécution de tâches par le membre d'équipage en fonction des mesures physiologiques et d'interaction du membre d'équipage avec l'aéronef (12),
le module de détermination d'état opérationnel d'équipage (62) étant propre à calculer l'état cognitif effectif du membre d'équipage en fonction des états cognitifs locaux et des états cognitifs globaux déterminés respectivement par le module de détermination d'états cognitifs globaux (60) et par le module de détermination d'états cognitifs locaux (58) et à comparer chaque état cognitif local ou global du membre d'équipage avec l'état cognitif local ou global théorique de référence du membre d'équipage déterminé par le module (44) de détermination de ressources équipage sur la base du modèle de ressources.

2. Système (10) selon la revendication 1, dans lequel le module de détermination d'état opérationnel (62) est propre à passer l'indicateur d'aptitude entre un état d'aptitude du membre d'équipage à effectuer la ou les tâches de la liste de tâches affectée au membre d'équipage et un état d'inaptitude du membre d'équipage à effectuer la ou les tâches de la liste de tâches affectée au membre d'équipage, en fonction d'une comparaison entre l'état cognitif effectif du membre d'équipage et l'état cognitif théorique du membre d'équipage.

3. Système (10) selon la revendication 2, comprenant un module (64) de reconfiguration de tâches de mission, propre à modifier la liste de tâches à effectuer par le membre d'équipage lorsque l'indicateur d'aptitude passe dans l'état d'inaptitude du membre d'équipage à effectuer la ou les tâches de la liste de tâches affectée au membre d'équipage.

4. Système (10) selon la revendication 3, dans lequel le module de reconfiguration de tâches (64) est propre à supprimer ou/et reporter une tâche à effectuer, à effectuer une tâche à la place du membre d'équipage, à supprimer des informations données au membre d'équipage, ou/et à remplacer les informations données au membre d'équipage par d'autres informations.

5. Système (10) selon l'une quelconque des revendications précédentes, dans lequel les états cognitifs globaux sont choisis parmi un niveau de charge mentale, un niveau d'engagement, un niveau de somnolence, un niveau d'hypoxie, et/ou un niveau de stress du membre d'équipage.

6. Système (10) selon l'une quelconque des revendications précédentes dans lequel les états cognitifs locaux sont choisis parmi un niveau d'attention, un niveau de persévération, un niveau de tunnélisation visuelle, un niveau de tunnélisation auditive dans la réalisation d'une tâche.

7. Système (10) selon l'une quelconque des revendications précédentes, comprenant un module (55) de mesures d'états physiologiques à partir de capteurs de mesures de données physiologiques (54), en particulier à partir de capteurs de mesure de fréquence cardiaque, de diamètre pupillaire, le nombre de clignements, d'oxygénation du sang, d'ondes cérébrales, de potentiels évoqués, et à partir d'une base de données (56) d'états physiologiques basiques de l'équipage, le module de détermination d'états cognitifs globaux (60) déterminant les états cognitifs globaux à partir des mesures d'états physiologiques du module de mesures d'états physiologiques (55).

8. Système (10) selon l'une quelconque des revendications précédentes, comprenant un module (50) de mesure d'états d'interactions avec l'équipage, à partir de capteurs (52) de mesures d'interactions avec l'équipage, en particulier la position du regard, l'utilisation de commandes physiques, l'utilisation de tactile, et à partir d'un modèle d'interactions attendues sur la base de la liste de tâches déterminée par le module de détermination de tâches d'équipage en cours (40), le module de détermination d'états cognitifs locaux (58) déterminant les états cognitifs locaux à partir des mesures d'états d'interactions du module de mesures d'états d'interactions (50).

9. Système (10) selon la revendication 8, dans laquelle les états d'interactions sont choisis parmi au minimum un niveau d'attention, un niveau de performance, une stratégie de conduite des tâches.

10. Système (10) selon l'une quelconque des revendications précédentes comportant un module (46) d'indication de contexte, en fonction de mesures d'environnement de la mission, propre à définir au moins un indicateur de contexte, le module de détermination de ressources équipage (44) étant propre à déterminer l'état cognitif théorique de référence du membre d'équipage en fonction du ou de chaque indicateur de contexte.

11. Système (10) selon l'une quelconque des revendications précédentes, dans lequel le module de détermination de tâches d'équipage en cours (40) est propre à attribuer la liste des tâches au membre d'équipage en fonction d'une base de données (42) de profils équipage déterminant les caractéristiques de chaque membre d'équipage et avantageusement des capacités de prise en charge des tâches par l'aéronef, et en fonction des objectifs de la mission

12. Procédé de détermination d'un état opérationnel d'un équipage d'aéronef (12) habité ou non habité, en fonction d'un plan de tâches adaptatif comprenant les étapes suivantes :
- fourniture d'un système (10) de détermination selon l'une quelconque des revendications précédentes ;
- définition, par le module de détermination de tâches d'équipage en cours (40), à chaque instant lors d'une mission de l'aéronef (12), d'une liste de tâches à effectuer par au moins un membre d'équipage en fonction d'objectifs de mission actualisés, et d'un contexte instantané de mission déterminé à partir de mesures d'environnement de la mission ;
- détermination, par le module de détermination de ressources équipage (44), pour le ou pour chaque membre d'équipage, d'au moins un état cognitif théorique de référence du membre d'équipage en fonction de la liste de tâches affectée au membre d'équipage, en utilisant un modèle de ressources ;
- détermination par le module de détermination d'état opérationnel d'équipage (62), d'un indicateur d'aptitude du ou de chaque membre d'équipage à effectuer la ou les tâches de la liste de tâches affectée au membre d'équipage à partir d'un état cognitif effectif du membre d'équipage déterminé sur la base de mesures physiologiques sur le membre d'équipage et de mesures d'interaction du membre d'équipage avec l'aéronef (12), et à partir de l'état cognitif théorique de référence du membre d'équipage déterminé par le module de détermination de ressources équipage (44),
- détermination d'états cognitifs globaux du membre d'équipage en fonction des mesures physiologiques et d'interaction du membre d'équipage, par le module (60) de détermination d'états cognitifs globaux du membre d'équipage et
- détermination d'états cognitifs locaux de l'équipage, relatifs à l'exécution de tâches par le membre d'équipage en fonction des mesures physiologiques et d'interaction du membre d'équipage avec l'aéronef (12) par le module (58) de détermination d'états cognitifs locaux de l'équipage,
- calcul par le module de détermination d'état opérationnel d'équipage (62) de l'état cognitif effectif du membre d'équipage en fonction des états cognitifs locaux et des états cognitifs globaux déterminés respectivement par le module de détermination d'états cognitifs globaux (60) et par le module de détermination d'états cognitifs locaux (58) et comparaison de chaque état cognitif local ou global du membre d'équipage avec l'état cognitif local ou global théorique de référence du membre d'équipage déterminé par le module (44) de détermination de ressources équipage sur la base du modèle de ressources.

13. Procédé selon la revendication 12, comprenant le passage de l'indicateur d'aptitude entre un état d'aptitude du membre d'équipage à effectuer la ou les tâches de la liste de tâches affectée au membre d'équipage et un état d'inaptitude du membre d'équipage à effectuer la ou les tâches de la liste de tâches affectée au membre d'équipage en fonction d'une comparaison entre l'état cognitif effectif du membre d'équipage et l'état cognitif théorique du membre d'équipage

14. Procédé selon la revendication 13, comprenant une modification, par un module de reconfiguration de tâches de mission (64), de la liste de tâches à effectuer par le membre d'équipage lorsque l'indicateur d'aptitude passe dans l'état d'inaptitude du membre d'équipage à effectuer la ou les tâches de la liste de tâches.

## Patentansprüche

1. System (10) zum Bestimmen eines Betriebszustands einer Besatzung eines bemannten oder unbemannten Luftfahrzeugs (12) abhängig von einem adaptiven Aufgabenplan, das System (10) umfassend:
- ein Modul (40) zum Bestimmen von auszuführenden Aufgaben der Besatzung, das geeignet ist, um zu jedem Zeitpunkt während einer Mission des Luftfahrzeugs (12) eine Liste von Aufgaben zu definieren, die von mindestens einem Besatzungsmitglied abhängig von aktualisierten Missionszielen und einem momentanen Missionskontext durchzuführen sind, der anhand von Umgebungsmessungen der Mission bestimmt wird; **gekennzeichnet durch**:
- ein Modul (44) zum Bestimmen von Besatzungsressourcen, das geeignet ist, um für das oder jedes Besatzungsmitglied mindestens einen theoretischen kognitiven Referenzzustand des Besatzungsmitglieds abhängig von der Aufgabenliste, die dem Besatzungsmitglied zugewiesen ist, unter Verwendung eines Ressourcenmodells zu bestimmen;
- ein Modul (62) zum Bestimmen des Betriebszustands der Besatzung, das geeignet ist, um einen Fähigkeitsindikator des oder jedes Besatzungsmitglieds zum Durchführen der Aufgabe(n) der dem Besatzungsmitglied zugewiesenen Aufgabenliste anhand eines tatsächlichen kognitiven Zustands des Besatzungsmitglieds, der basierend auf physiologischen Messungen an dem Besatzungsmitglied und Messungen der Interaktion des Besatzungsmitglieds mit dem Luftfahrzeug (12) bestimmt wird, und anhand des theoretischen kognitiven Referenzzustands des Besatzungsmitglieds, der durch das Modul (44) zum Bestimmen der Besatzungsressourcen bestimmt wird, zu bestimmen,
- ein Modul (60) zum Bestimmen globaler kognitiver Zustände des Besatzungsmitglieds basierend auf physiologischen und Interaktionsmessungen des Besatzungsmitglieds, und
- ein Modul (58) zum Bestimmen lokaler kognitiver Zustände der Besatzung, das geeignet ist, lokale kognitive Zustände zu bestimmen, die sich auf die Ausführung von Aufgaben durch das Besatzungsmitglied beziehen, abhängig von physiologischen Messungen und der Interaktion des Besatzungsmitglieds mit dem Luftfahrzeug (12),
wobei das Modul (62) zum Bestimmen des Betriebszustands der Besatzung geeignet ist, um den tatsächlichen kognitiven Zustand des Besatzungsmitglieds basierend auf den lokalen kognitiven Zuständen und den globalen kognitiven Zuständen, die jeweils durch das Modul (60) zum Bestimmen globaler kognitiver Zustände und das Modul (58) zum Bestimmen lokaler kognitiver Zustände bestimmt werden, zu berechnen und jeden lokalen oder globalen kognitiven Zustand des Besatzungsmitglieds mit dem theoretischen lokalen oder globalen kognitiven Referenzzustand des Besatzungsmitglieds zu vergleichen, der durch das Modul (44) zum Bestimmen von Besatzungsressourcen basierend auf dem Ressourcenmodell bestimmt wird.

2. System (10) nach Anspruch 1, wobei das Modul (62) zum Bestimmen des Betriebszustands der Besatzung geeignet ist, um den Fähigkeitsindikator zwischen einem Zustand der Fähigkeit des Besatzungsmitglieds, die Aufgabe(n) der dem Besatzungsmitglied zugewiesenen Aufgabenliste durchzuführen, und einem Zustand der Unfähigkeit des Besatzungsmitglieds, die Aufgabe(n) der dem Besatzungsmitglied zugewiesenen Aufgabenliste durchzuführen, basierend auf einem Vergleich zwischen dem tatsächlichen kognitiven Zustand des Besatzungsmitglieds und dem theoretischen kognitiven Zustand des Besatzungsmitglieds überzugehen.

3. System (10) nach Anspruch 2, umfassend ein Modul (64) zum Neukonfigurieren von Missionsaufgaben, das geeignet ist, um die Liste der von dem Besatzungsmitglied durchzuführenden Aufgaben zu ändern, wenn der Fähigkeitsindikator in den Zustand übergeht, in dem das Besatzungsmitglied nicht in der Lage ist, die Aufgabe(n) der dem Besatzungsmitglied zugewiesenen Aufgabenliste durchzuführen.

4. System (10) nach Anspruch 3, wobei das Aufgabenneukonfigurationsmodul (64) geeignet ist, um eine durchzuführende Aufgabe zu löschen oder/und zu verschieben, eine Aufgabe anstelle des Besatzungsmitglieds durchzuführen, Informationen, die dem Besatzungsmitglied gegeben werden, zu löschen oder/und die dem Besatzungsmitglied gegebenen Informationen durch andere Informationen zu ersetzen.

5. System (10) nach einem der vorherigen Ansprüche, wobei die globalen kognitiven Zustände ausgewählt sind aus einem mentalen Belastungsgrad, einem Engagementgrad, einem Schläfrigkeitsgrad, einem Hypoxiegrad und/oder einem Stressgrad des Besatzungsmitglieds.

6. System (10) nach einem der vorherigen Ansprüche, wobei die lokalen kognitiven Zustände ausgewählt sind aus einem Aufmerksamkeitsgrad, einem Perseverationsgrad, einem visuellen Tunnelinggrad, einem auditiven Tunnelinggrad bei der Durchführung einer Aufgabe.

7. System (10) nach einem der vorherigen Ansprüche, umfassend ein Modul (55) zum Messen physiologischer Zustände anhand von Messsensoren physiologischer Daten (54), insbesondere anhand von Messsensoren der Herzfrequenz, des Pupillendurchmessers, der Anzahl von Blinzeln, des Sauerstoffgehalts des Bluts, von Gehirnwellen, evozierten Potentialen, und aus einer Datenbank (56) von grundlegenden physiologischen Zuständen der Besatzung, wobei das Modul (60) zum Bestimmen von globalen kognitiven Zuständen die globalen kognitiven Zustände anhand der Messungen der physiologischen Zustände des Moduls (55) zum Messen physiologischer Zustände bestimmt.

8. System (10) nach einem der vorherigen Ansprüche, umfassend ein Modul (50) zum Messen von Besatzungsinteraktionszuständen, anhand von Sensoren (52) zum Messen von Besatzungsinteraktionen, insbesondere der Blickposition, der Verwendung von physischen Steuerungen, der Verwendung von taktilen, und anhand eines Modells von erwarteten Interaktionen basierend auf der Aufgabenliste, die durch das Modul (40) zum Bestimmen der aktuellen Besatzungsaufgaben bestimmt wird, wobei das Modul (58) zum Bestimmen lokaler kognitiver Zustände die lokalen kognitiven Zustände anhand der Interaktionszustandsmessungen des Moduls (50) zum Messen von Interaktionszuständen bestimmt.

9. System (10) nach Anspruch 8, wobei die Interaktionszustände ausgewählt sind aus mindestens einem Aufmerksamkeitsgrad, einem Leistungsgrad, einer Aufgabenerledigungsstrategie.

10. System (10) nach einem der vorherigen Ansprüche, umfassend ein Modul (46) zur Kontextangabe abhängig von Umgebungsmessungen der Mission, das geeignet ist, um mindestens einen Kontextindikator zu definieren, wobei das Modul zum Bestimmen der Besatzungsressourcen (44) geeignet ist, um den theoretischen kognitiven Referenzzustand des Besatzungsmitglieds abhängig von dem oder jedem Kontextindikator zu bestimmen.

11. System (10) nach einem der vorherigen Ansprüche, wobei das Modul zum Bestimmen der aktuellen Besatzungsaufgaben (40) geeignet ist, um die Liste der Aufgaben dem Besatzungsmitglied abhängig von einer Datenbank (42) von Besatzungsprofilen, die die Eigenschaften jedes Besatzungsmitglieds und vorteilhafterweise die Fähigkeiten zur Übernahme der Aufgaben durch das Luftfahrzeug bestimmt, und abhängig von den Zielen der Mission zuzuweisen.

12. Verfahren zum Bestimmen eines Betriebszustands einer Besatzung eines bemannten oder unbemannten Luftfahrzeugs (12) abhängig von einem adaptiven Aufgabenplan, umfassend die folgenden Schritte:
- Bereitstellen eines Systems (10) zum Bestimmen nach einem der vorherigen Ansprüche;
- Definieren, durch das Bestimmungsmodul aktueller Aufgaben der Besatzung (40), zu jedem Zeitpunkt während einer Mission des Luftfahrzeugs (12), einer Liste von Aufgaben, die von mindestens einem Besatzungsmitglied abhängig von aktualisierten Missionszielen und einem momentanen Missionskontext durchzuführen sind, der anhand von Umgebungsmessungen der Mission bestimmt wird;
- Bestimmen, durch das Besatzungsressourcen-Bestimmungsmodul (44), für das oder jedes Besatzungsmitglied mindestens eines theoretischen kognitiven Referenzzustands des Besatzungsmitglieds abhängig von der Aufgabenliste, die dem Besatzungsmitglied zugewiesen ist, unter Verwendung eines Ressourcenmodells;
- Bestimmen, durch das Bestimmungsmodul eines operativen Zustands der Besatzung (62), eines Indikators für die Fähigkeit des oder jedes Besatzungsmitglieds zum Durchführen der Aufgabe(n) der dem Besatzungsmitglied zugewiesenen Aufgabenliste anhand eines tatsächlichen kognitiven Zustands des Besatzungsmitglieds, der basierend auf physiologischen Messungen an dem Besatzungsmitglied und Messungen der Interaktion des Besatzungsmitglieds mit dem Luftfahrzeug (12) bestimmt wird, und anhand des theoretischen kognitiven Referenzzustands des Besatzungsmitglieds, der durch das Besatzungsressourcen-Bestimmungsmodul (44) bestimmt wird,
- Bestimmen von globalen kognitiven Zuständen des Besatzungsmitglieds basierend auf physiologischen und Interaktionsmessungen des Besatzungsmitglieds durch das Modul (60) zum Bestimmen von globalen kognitiven Zuständen des Besatzungsmitglieds, und
- Bestimmen lokaler kognitiver Zustände der Besatzung, die sich auf die Ausführung von Aufgaben durch das Besatzungsmitglied beziehen, basierend auf physiologischen und Interaktionsmessungen des Besatzungsmitglieds mit dem Luftfahrzeug (12) durch das Modul (58) zum Bestimmen lokaler kognitiver Zustände der Besatzung,
- Berechnen des tatsächlichen kognitiven Zustands des Besatzungsmitglieds durch das Bestimmungsmodul eines operativen Zustands der Besatzung (62) basierend auf den lokalen kognitiven Zuständen und den globalen kognitiven Zuständen, die jeweils durch das Modul (60) zum Bestimmen globaler kognitiver Zustände und das Modul (58) zum Bestimmen lokaler kognitiver Zustände bestimmt werden, und Vergleichen jedes lokalen oder globalen kognitiven Zustands des Besatzungsmitglieds mit dem theoretischen lokalen oder globalen kognitiven Referenzzustand des Besatzungsmitglieds, der durch das Modul (44) zum Bestimmen von Besatzungsressourcen basierend auf dem Ressourcenmodell bestimmt wird.

13. Verfahren nach Anspruch 12, umfassend ein Übergehen des Fähigkeitsindikators zwischen einem Zustand der Fähigkeit des Besatzungsmitglieds, die Aufgabe(n) der dem Besatzungsmitglied zugewiesenen Aufgabenliste durchzuführen, und einem Zustand der Unfähigkeit des Besatzungsmitglieds, die Aufgabe(n) der dem Besatzungsmitglied zugewiesenen Aufgabenliste durchzuführen, basierend auf einem Vergleich zwischen dem tatsächlichen kognitiven Zustand des Besatzungsmitglieds und dem theoretischen kognitiven Zustand des Besatzungsmitglieds.

14. Verfahren nach Anspruch 13, umfassend eine Änderung der Liste der von dem Besatzungsmitglied durchzuführenden Aufgaben durch ein Missionsaufgaben-Neukonfigurationsmodul (64), wenn der Fähigkeitsindikator in den Zustand übergeht, dass das Besatzungsmitglied nicht in der Lage ist, die Aufgabe(n) der Aufgabenliste durchzuführen.

## Claims

1. A system (10) for determining an operational state of an aircrew of a manned or unmanned aircraft (12) according to an adaptive task plan, the system (10) comprising:
- a module (40) for determining crew tasks to be carried out, capable of defining, at each moment during a mission of the aircraft (12), a list of tasks to be carried out by at least one crew member as a function of updated mission objectives, and of an instantaneous mission context determined from measurements of the mission environment; **characterized by** :
- a crew resource determination module (44), adapted to determine for the or each crew member at least one theoretical baseline crew member cognitive state according to the task list assigned to the crew member, using a resource model;
- a crew operational state determination module (62), adapted to determine an indicator of the fitness of the or each crew member to perform the task(s) of the task list assigned to the crew member, from an actual crew member cognitive state determined on the basis of physiological measurements on the crew member and measurements of the crew member's interaction with the aircraft (12), and from the theoretical baseline crew member cognitive state determined by the crew resource determination module (44).
- a module (60) for determining overall cognitive states of the crew member as a function of the physiological and interaction measurements of the crew member and
- a module (58) for determining local cognitive states of the crew, adapted to determine local cognitive states related to the execution of tasks by the crew member as a function of the crew member's physiological measurements and aircraft (12) interaction measurements,
the crew operational state determination module (62) being adapted to calculate the actual crew member cognitive state as a function of the local cognitive states and the overall cognitive states determined by the overall cognitive state determination module (60) and the local cognitive state determination module (58), respectively, and to compare each local or overall crew member cognitive state with the theoretical baseline local or overall crew member cognitive state determined by the crew resource determination module (44) based on the resource model.

2. The system (10) according to claim 1, wherein the operational state determination module (62) is adapted to transition the fitness indicator between a state of fitness of the crew member to perform the task(s) of the task list assigned to the crew member and a state of unfitness of the crew member to perform the task(s) of the task list assigned to the crew member, based on a comparison between the actual crew member cognitive state and the theoretical crew member cognitive state.

3. The system (10) according to claim 2, comprising a mission task reconfiguration module (64), suitable for modifying the task list to be performed by the crew member when the fitness indicator changes to the state of unfitness of the crew member to perform the task(s) of the task list assigned to the crew member.

4. The system (10) according to claim 3, wherein the task reconfiguration module (64) is suitable for deleting or/and postponing a task to be performed, performing a task instead of the crew member, deleting information given to the crew member, and/or replacing the information given to the crew member with other information.

5. The system (10) according to any of the preceding claims, wherein the overall cognitive states are selected from a level of mental load, a level of engagement, a level of sleepiness, a level of hypoxia, and/or a level of stress of the crew member.

6. The system (10) according to any of the preceding claims, wherein the local cognitive states are selected from a level of attention, a level of perseveration, a level of visual tunnelling, or a level of auditory tunnelling in the performance of a task.

7. The system (10) according to any of the preceding claims, comprising a module (55) for measuring physiological states based on sensors for measuring physiological data (54), in particular based on sensors for measuring heart rate, pupil diameter, number of blinks, blood oxygenation, brain waves, evoked potentials, and based on a database (56) of basic physiological states of the crew, the module for determining overall cognitive states determining the overall cognitive states (60) from the physiological state measurements of the physiological state measurement module (55).

8. The system (10) according to any of the preceding claims, comprising a module (50) for measuring crew interaction states, based on sensors (52) for measuring crew interaction states, in particular gaze position, the use of physical commands, and the use of touch, and based on a model of expected interactions based on the list of tasks determined by the module (40) for determining current crew tasks, the local cognitive state determination module (58) determining local cognitive states from the interaction state measurements of the module for measuring interaction states (50).

9. The system (10) according to claim 8, wherein the interaction states are selected from at least one level of attention, one level of performance, and one task completion strategy.

10. The system (10) according to any of the preceding claims, comprising a context indication module (46), which based on mission environment measurements is adapted to define at least one context indicator, the crew resource determination module (44) being adapted to determine the theoretical baseline crew member cognitive state based on the or each context indicator.

11. The system (10) according to any of the preceding claims, wherein the current crew task determination module (40) is adapted to assign the list of tasks to the crew member according to a database (42) of crew profiles determining the characteristics of each crew member and advantageously the task handling capabilities of the aircraft, and according to the mission objectives.

12. A method of determining an operational state of an aircrew of a manned or unmanned aircraft (12), based on an adaptive task plan comprising the following steps:
- providing a determination system (10) according to any one of the preceding claims;
- defining, by the module (40) for determining current crew tasks, at each moment during a mission of the aircraft (12), of a list of tasks to be carried out by at least one crew member as a function of updated mission objectives, and of an instantaneous mission context determined from environmental measurements of the mission;
- determination, by the crew resource determination module (44), for the or each crew member, of at least one theoretical baseline crew member cognitive state based on the task list assigned to the crew member, using a resource model;
- determination, by the crew operational state determination module (62), adapted to determine an indicator of the fitness of the or each crew member to perform the task(s) of the task list assigned to the crew member, from an actual crew member cognitive state determined on the basis of physiological measurements on the crew member and measurements of the crew member's interaction with the aircraft (12), and from the theoretical baseline crew member cognitive state determined by the crew resource determination module (44);
- determination of the crew member's overall cognitive states as a function of the crew member's physiological and interaction measurements, by the module (60) for determining the crew member's overall cognitive states, and
- determination of local cognitive states of the crew, relative to the execution of tasks by the crew member as a function of the crew member's physiological and interaction measurements with the aircraft (12) by the module (58) for determining local cognitive states of the crew;
- calculation by the crew operational state determination module (62) of the crew member's actual cognitive state as a function of the local cognitive states and global cognitive states determined by the global cognitive state determination module (60) and local cognitive state determination module (58) respectively, and comparison of each local or global cognitive state of the crew member with the crew member's theoretical reference local or global cognitive state determined by the crew resource determination module (44) on the basis of the resource model.

13. The method according to claim 12, comprising the transition of the fitness indicator between a state of fitness of the crew member to perform the task(s) on the crew member's assigned task list and a state of unfitness of the crew member to perform the task(s) on the crew member's assigned task list based on a comparison between the actual crew member cognitive state and the theoretical crew member cognitive state.

14. The method according to claim 13, comprising a modification, by a mission task reconfiguration module (64), of the task list to be performed by the crew member when the fitness indicator changes to the state of unfitness of the crew member to perform the task(s) on the task list.
